# EUROPEAN PATENT APPLICATION

(11) **EP 4 652 989 A1**
(43) Date of publication of application: **26.11.2025**
(21) Application number: 25176581.4
(22) Date of filing: 15.05.2025
(51) Int. Cl.: A61K 9/16, A61K 9/20, A61K 31/00

(54) **COATED TENOFOVIR ALAFENAMIDE**

(30) Priority: 21.05.2024 GB 202407194
(71) Applicant: Zentiva K.S., 102 37 Praha 10 (CZ)
(72) Inventor: OBADALOVA, Iva, 198 00 Praha 9 (CZ); ZVÁTORA, Pavel, 198 00 Praha 9 (CZ); PETRIK, Jakub, 148 00 Praha 4 (CZ)
(74) Representative: Reddie & Grose LLP

(57) **Abstract**

The present invention relates to a polymer-coated tenofovir alafenamide. The present invention also relates to a pharmaceutical composition comprising a polymer-coated tenofovir alafenamide, and a method of preparing a polymer-coated tenofovir alafenamide.

## Description

### Field of the Invention

The present invention relates to a polymer-coated tenofovir alafenamide. The present invention also relates to a pharmaceutical composition comprising a polymer-coated tenofovir alafenamide, and a method of preparing a polymer-coated tenofovir alafenamide.

A polymer-coated tenofovir alafenamide or a pharmaceutical composition comprising the polymer-coated tenofovir alafenamide may also be used in the prevention and/or treatment of hepatitis B or HIV.

### Background

Tenofovir alafenamide (TAF) is a nucleotide reverse transcriptase inhibitor which has the following chemical structure:

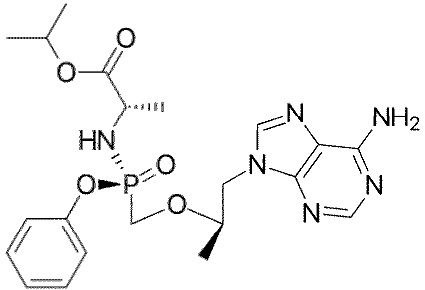

It is a phosphonamidate prodrug of tenofovir, and has activity that is specific to hepatitis B virus (HBV) and human immunodeficiency virus (HIV).

A number of combination products comprising TAF for the treatment of HIV are currently on the market, including TAF/emtricitabine; TAF/emtricitabine/elvitegravir/cobicistat; TAF/emtricitabine/rilpivirine; TAF/emtricitabine/bictegravir; TAF/emtricitabine/dolutegravir and TAF/emtricitabine/darunavir/cobicistat.

Whilst TAF is more stable in human blood plasma than the first approved oral prodrug of tenofovir i.e. tenofovir disoproxil fumarate (TDF), a need still remains to provide TAF in a more stable form. More specifically, TAF, particularly in the free base form, can be sensitive to moisture. Therefore it would be desirable to find an effective way to prevent degradation of TAF, especially in humid conditions.

### Summary of Invention

In a first aspect, the present invention provides a polymer-coated tenofovir alafenamide.

In a second aspect, the present invention provides a pharmaceutical composition comprising a polymer-coated tenofovir alafenamide.

In a third aspect, the present invention provides a method of preparing a polymer-coated tenofovir alafenamide comprising the steps of:
a) coating tenofovir alafenamide with a solution comprising a polymer and a solvent; and
b) removing the solvent to obtain the polymer-coated tenofovir alafenamide.

In a fourth aspect, the present invention provides a polymer-coated tenofovir alafenamide produced by a fluid bed granulation process.

In a fifth aspect, the present invention provides a polymer-coated tenofovir alafenamide for use in the prevention and/or treatment of hepatitis B or HIV. The present invention also provides a pharmaceutical composition comprising a polymer-coated tenofovir alafenamide for use in the prevention and/or treatment of hepatitis B or HIV

In a sixth aspect, the present invention provides a method of preventing and/or treating hepatitis B or HIV, comprising administering an effective amount of a polymer-coated tenofovir alafenamide to a patient in need thereof.

In a seventh aspect, the present invention provides a use of a polymer-coated tenofovir alafenamide in the manufacture of a medicament for the prevention and/or treatment of hepatitis B or HIV.

Other features, embodiments and advantages will be apparent from the description and the claims.

### Description of the Figures

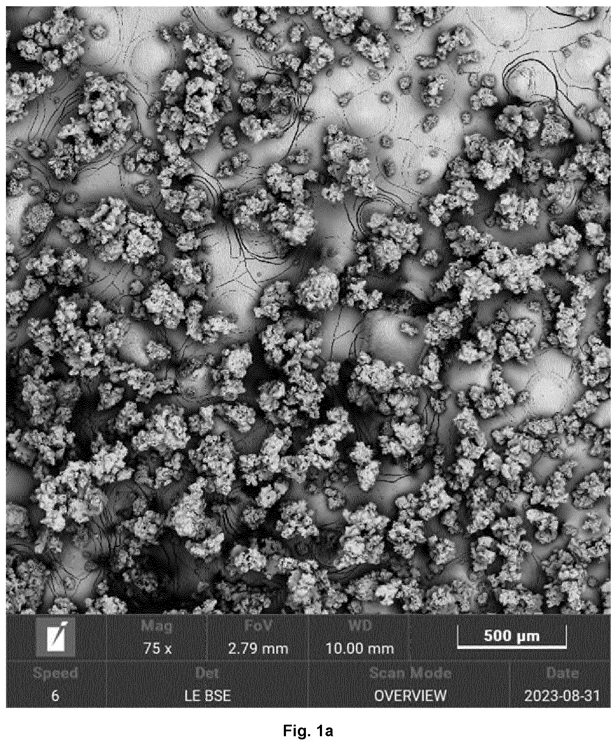

Fig. 1a: Scanning electron microscope (SEM) photo of polymer-coated tenofovir alafenamide prepared from micronized tenofovir alafenamide.

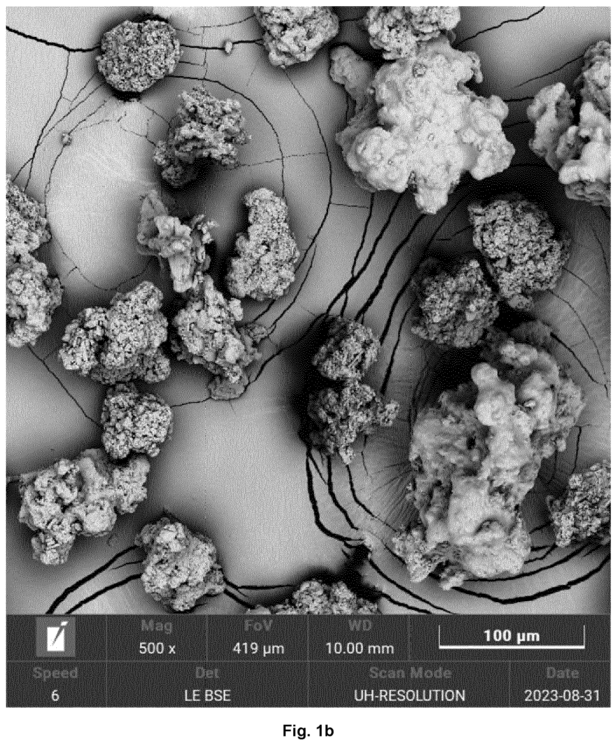

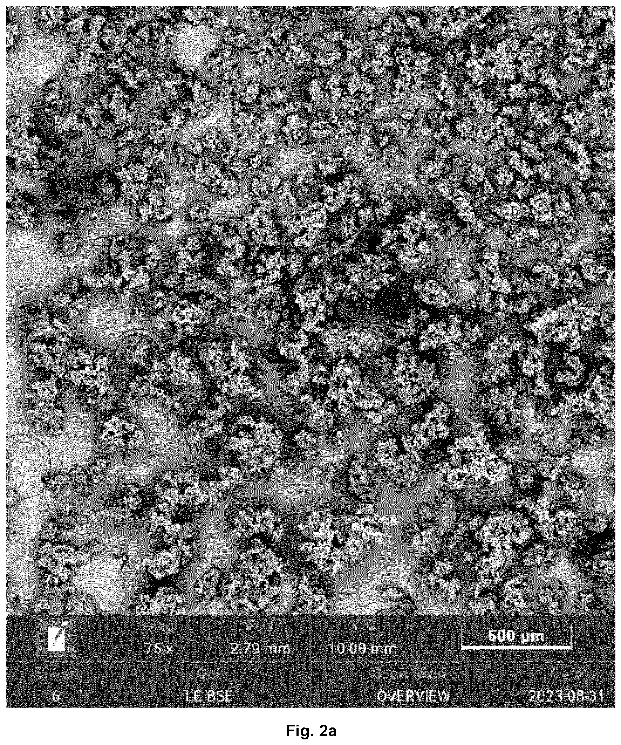

Fig. 1b: Magnified scanning electron microscope (SEM) photo of polymer-coated tenofovir alafenamide prepared from micronized tenofovir alafenamide as depicted in Fig. 2a.
Fig. 2a: Scanning electron microscope (SEM) photo of polymer-coated tenofovir alafenamide prepared from non-micronized tenofovir alafenamide.

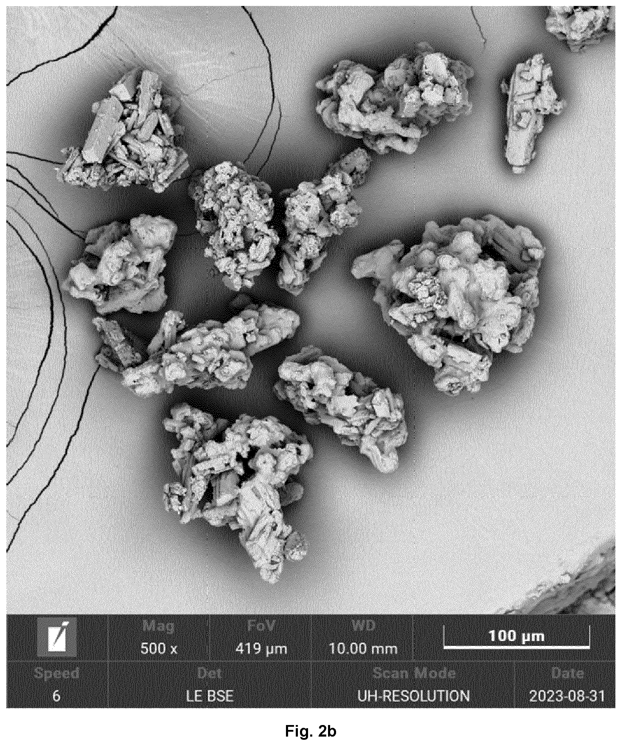

Fig. 2b: Magnified scanning electron microscope (SEM) photo of polymer-coated tenofovir alafenamide prepared from non-micronized tenofovir alafenamide as depicted in Fig. 3a.

### Detailed Description

A first aspect of the present invention provides a polymer-coated tenofovir alafenamide.

The tenofovir alafenamide may be provided in a free base form or as a pharmaceutically acceptable salt. The term "salt" as used herein refers to a salt of tenofovir alafenamide that is derived from suitable inorganic and organic acids and bases. Examples of salts of a basic group include those formed with inorganic acids such as hydrochloric acid, hydrobromic acid, phosphoric acid, sulfuric acid and perchloric acid or with organic acids such as trifluoroacetic acid, acetic acid, oxalic acid, maleic acid, tartaric acid, citric acid, succinic acid or malonic acid or by using other methods used in the art such as ion exchange. Other salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, hemifumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁-C₄ alkyl)₄ salts. Representative alkali or alkaline earth metal salts include sodium, lithium, potassium, calcium, magnesium, and the like. Further salts include, when appropriate, nontoxic ammonium, quaternary ammonium, and amine cations formed using counterions such as halide, hydroxide, carboxylate, sulfate, phosphate, nitrate, alkyl sulfonate and aryl sulfonate. In a preferred embodiment, tenofovir alafenamide is provided in a free base form or as a fumarate or hemifumarate salt form. In a more preferred embodiment, tenofovir alafenamide is provided in a free base form.

In some aspects, tenofovir alafenamide may exist in an unsolvated form as well as the solvated form, including the hydrated form. "Hydrate" refers to a complex formed by combination of water molecules with molecules or ions of the solute. "Solvate" refers to a complex formed by combination of solvent molecules with molecules or ions of the solute. The solvent may be an organic compound, an inorganic compound, or a mixture of both. Solvate is meant to include hydrate. Some examples of solvents include, but are not limited to, methanol, acetonitrile, N,N-dimethylformamide, tetrahydrofuran, dimethylsulfoxide, and water. In general, the solvated forms are equivalent to unsolvated forms and are encompassed within the scope of the present invention.

The tenofovir alafenamide may be provided in a micronized or non-micronized form, and more specifically as particles in a micronized or non-micronized form. The term "micronized" as used herein refers to particles having an average particle diameter of less than about 200 µm, preferably less than about 150 µm and more preferably less than about 100 µm. In a more preferred embodiment, the particles may have an average particle diameter between about 10 to about 200 µm, preferably between about 20 to about 200 µm, preferably between about 20 to about 150 µm, and preferably between about 20 to about 100 µm. The term "an average particle diameter" as used herein refers to a particle diameter of 50% cumulative percentage (D₅₀) on volume-based measurement of powder particle, which may be measured on the mass with a commercially available laser diffraction type particle size distribution measuring device and sonic wave vibration type sieving analyzer.

Alternatively, particles may be characterized by having D₉₀ particle size of less than about 200µm, preferably less than about 150µm and more preferably less than about 100µm. D₉₀ may be defined as the particle diameter of accumulated 90% based on volume by laser diffraction scattering particle size distribution measurement. Particle size distribution may be determined by laser diffraction. For example, the particle size distribution, including the D₉₀ value may be determined by an analyser such as the Malvern Mastersizer Hydro 2000MU.

In a first aspect of the present invention, the tenofovir alafenamide may be coated with a polymer. The term "coated" as used herein refers to tenofovir alafenamide that is at least partly covered by a polymer. In certain embodiments, the tenofovir alafenamide may be partially covered or may be fully covered by a polymer. In an embodiment, solely the tenofovir alafenamide may be coated with the polymer. Accordingly, a polymer-coated tenofovir alafenamide may be distinguished from compositions or dosage forms comprising tenofovir alafenamide and an outer/protective/film coating. A polymer-coated tenofovir alafenamide may also be distinguished from compositions comprising tenofovir alafenamide and a polymer as separate ingredients within the composition.

In an embodiment, polymer-coated tenofovir alafenamide may be in the form of individual particles of tenofovir alafenamide coated with a polymer. In another embodiment, individual particles of tenofovir alafenamide may be coated with a polymer and then formed into agglomerates. In yet a further embodiment, agglomerates of tenofovir alafenamide may be formed from individual particles which may then be coated with a polymer. In a further embodiment, tenofovir alafenamide may be present in a combination of any of these forms (e.g. as a mixture of individual particles and agglomerates).

The polymer used to coat the tenofovir alafenamide may be any pharmaceutically acceptable polymer.

The polymer may be selected from the group consisting of hydrophilic or hydrophobic polymers comprising one or more of polyvinyl acetate, cellulose acetate, cellulose acetate butyrate, cellulose acetate propionate, ethyl cellulose, a fatty acid, a fatty acid ester, an alkyl alcohol, a wax, xanthan gum, gellan gum, shellac, rosin, zein (prolamine from corn), povidone, kollidon SR (polyvinyl acetate and povidone), a poly(meth)acrylate, poly(ethylene oxide), polyuronic acid salts, cellulose ethers (e.g. methocel E5), xanthan gum, tragacanth gum, gum karaya, guar gum, acacia, gellan gum locust bean gum, alkali metal salts of alginic acid or pectic acid, sodium alginate, potassium alginate, ammonium alginate, polyethylene oxide, carbomer homopolymer, hydroxypropyl cellulose, hydroxy ethyl cellulose, methylhydroxyethylcellulose, hydroxypropyl methyl cellulose (hypromellose) (e.g., type 2208, type 2910, methocel K15M, methocel E15LV), carboxyvinyl polymers, polyvinyl alcohol, gelatin, shellac, methacrylic acid copolymer type C - NF, cellulose butyrate phthalate, cellulose hydrogen phthalate, cellulose propionate phthalate, polyvinyl acetate phthalate (PVAP), cellulose acetate phthalate (CAP), cellulose acetate trimellitate (CAT), hydroxypropyl methylcellulose phthalate, hydrogenated castor oil, stearic acid, hydrogenated vegetable oil, glyceryl behenate, glyceryl monostearate, hydroxypropyl methylcellulose acetate, dioxypropyl methylcellulose succinate, carboxymethyl ethyl cellulose (CMEC), hydroxypropyl methylcellulose acetate succinate (HPMCAS), and acrylic acid polymers and copolymers such as methyl acrylate, ethyl acrylate, methyl methacrylate and/or ethyl methacrylate with copolymers of acrylic and methacrylic acid esters (Eudragit NE, Eudragit RL, Eudragit RS), Carbomer (e.g., Carbomer 971P) and combinations thereof.

In a preferred embodiment, the polymer may be a non-ionizable pharmaceutically acceptable polymer. The term "non-ionizable" as used herein refers to a polymer that possesses substantially no ionizable functional groups.

The non-ionizable pharmaceutically acceptable polymer may be selected from the group consisting of derivates of cellulose (namely ethyl cellulose, hydroxyethyl cellulose, methylhydroxyethylcellulose, hypromellose), cellulose ether (e.g. methocel), polyvinyl alcohol, povidone, shellac, gelatin and combinations thereof. In a preferred embodiment, the polymer may be a derivate of cellulose, and more preferably cellulose ether.

The polymer may be present in an amount of about 0.5% to about 50%, preferably about 1% to about 45%, preferably about 1% to about 40%, preferably about 2% to about 35%, preferably about 3% to about 30%, preferably about 4% to about 25%, preferably about 5% to about 25%, preferably about 6% to about 24%, preferably about 7% to about 23%, preferably about 8% to about 22%, preferably about 9% to about 21%, preferably about 10% to about 20%, by weight based on the total weight of tenofovir alafenamide. In an embodiment, the coating may be present in an amount of about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%,or about 25% by weight based on the total weight of tenofovir alafenamide.

The weight ratio between the polymer and tenofovir alafenamide may be between about 1:200 and about 1:2, preferably between about 1:100 and about 1:2, preferably between about 1:50 and about 2:5, preferably between about 1:40 and about 2:5, preferably between about 1:30 and about 1:3, preferably between about 1:25 and about 1:3, preferably between about 1:20 and about 1:4, preferably between about 1:15 and about 1:4, and preferably between about 1:10 and about 1:5.

It has surprisingly been found that a polymer-coated tenofovir alafenamide was more stable and less prone to degradation compared to non-coated tenofovir alafenamide. This effect was particularly noticeable when tenofovir alafenamide in the free base form was used.

A second aspect of the present invention provides a pharmaceutical composition comprising a polymer-coated tenofovir alafenamide.

The polymer-coated tenofovir alafenamide may be the same as that referred to in relation to the first aspect of the invention.

The pharmaceutical composition may comprise tenofovir alafenamide in an amount of about 1% to about 15%, preferably about 1% to about 10%, preferably about 1% to about 9%, preferably about 1% to about 8%, preferably about 1% to about 7%, and preferably about 2% to about 7%, by weight based on the total weight of the composition. In an embodiment, tenofovir alafenamide may be present in an amount of about 1%, about 2%, about 3%, about 4%, about 5%, about 6%, about 7%, about 8%, about 9%, about 10%, about 11%, about 12%, about 13%, about 14%, or about 15%, by weight based on the total weight of the composition.

The pharmaceutical composition according to the present invention may comprise about 10 mg to about 30 mg, or preferably about 10 mg to about 25 mg of tenofovir alafenamide. In an embodiment, the composition may comprise about 10 mg, about 15 mg, about 20 mg, or about 25 mg, preferably about 10 mg or 25 mg of tenofovir alafenamide.

As used herein, and in the absence of a specific reference to a particular pharmaceutically acceptable salt and/or solvate of tenofovir alafenamide, any dosages, whether expressed in e.g. milligrams or as a % by weight, should be taken as referring to the amount of tenofovir alafenamide in free base form. The amount expressed in milligrams or as a % by weight refers to the amount of tenofovir alafenamide in free base form without the polymer coating.

The pharmaceutical composition may comprise polymer-coated tenofovir alafenamide in an amount of about 1% to about 60%, preferably about 1% to about 50%, preferably about 1% to about 40%, preferably about 2% to about 30%, preferably about 2% to about 25%, preferably about 2% to about 20%, preferably about 2% to about 15%, and preferably about 3% to about 10%, by weight based on the total weight of the composition. In an embodiment, tenofovir alafenamide may be present in an amount of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight based on the total weight of the composition.

The pharmaceutical composition according to the second aspect of the present invention may include one or more pharmaceutically acceptable carriers or excipients. Pharmaceutically acceptable carriers or excipients may include binders, fillers, glidants, lubricants, disintegrants and plasticizers.

The binder may be selected from the group consisting of hydroxymethyl cellulose, hydroxypropylcellulose, polyvinylpyrrolidone, calcium carbonate, dicalcium phosphate, carbomers, cellulose acetate phthalates, copovidone, hydroxypropyl methyl cellulose, ethylene glycol and vinyl glycol grafted copolymer, isomalt, poloxamer, polyethylene oxide, polymethacrylates, and combinations thereof.

The binder may be present in an amount of about 0.1% to about 20%, preferably about 0.5% to about 15%, and preferably about 1% to about 10%, by weight based on the total weight of the composition.

The filler may be selected from the group consisting of calcium carbonate, calcium phosphate, dibasic calcium phosphate, calcium silicate, tribasic calcium sulfate, calcium carboxymethylcellulose and salts thereof, cellulose, dextrin derivatives, dextrin, dextrose, fructose, isomalt, kaolin, lactitol, lactose, magnesium carbonate, magnesium oxide, maltitol, maltodextrins, maltose, mannitol, microcrystalline cellulose, sodium bicarbonate, sodium carbonate, sorbitol, starch, sucrose, sugar, xylitol, and combinations thereof. The filler may preferably be microcrystalline cellulose.

The filler may be present in an amount of about 20% to about 65%, preferably about 20% to about 60%, preferably about 25% to about 60%, preferably about 30% to about 60%, preferably about 30% to about 55%, and preferably about 30% to about 50%, by weight based on the total weight of the composition.

The glidant may be selected from the group consisting of silica, fumed silica, silicified cellulose, sodium stearate, magnesium aluminium silicate, pyrogenic silica, hydrated sodium silioaluminate, cellulose, calcium phosphate, sodium lauryl sulfate, pregelatinized starch, talc, and physical or co-processed combinations thereof.

The glidant may be present **in** an amount of about 0.1% to about 10%, preferably about 0.5% to about 5%, and preferably about 1% to about 3%, by weight based on the total weight of the composition.

The lubricant may be selected from the group consisting of agar, calcium stearate, ethyl oleate, ethyl laureate, glycerin, glyceryl behenate, glyceryl palmitostearate, hydrogenated vegetable oil, magnesium oxide, magnesium stearate, mannitol, poloxamer, glycols, sodium benzoate, sodium lauryl sulfate, sodium stearyl fumarate, sorbitol, stearic acid, talc, zinc stearate, and combinations thereof. The lubricant may preferably be magnesium stearate.

The lubricant may be present **in** an amount of about 0.1% to about 10%, preferably about 0.5% to about 5%, and preferably about 1% to about 3%, by weight based on the total weight of the composition.

The disintegrant may be selected from the group consisting of agar-agar, algins, calcium carbonate, carboxymethylcellulose and salts thereof, cellulose, clays, corn starch, croscarmellose sodium (Ac-Di-Sol), crospovidone, gums, methyl cellulose, polacrilin potassium, sodium alginate, cross-linked polyvinylpyrrolidone, sodium starch glycolate, starch, and combinations thereof. The disintegrant may preferably be croscarmellose sodium.

The disintegrant may be present **in** an amount of about 0.01% to about 15%, preferably about 0.05% to about 12%, and preferably about 0.1% to about 10%, by weight based on the total weight of the composition.

The plasticizer may be selected from the group consisting of propylene glycol, phthalates, polyethylene glycols, sebacates, or citrates such as dibutyl phthalate, diethyl phthalate, dibutyl sebecate, triethyl citrate, acetyl tributyl citrate, polyethylene glycol and combinations thereof.

The plasticizer may be present **in** an amount of about 0.1% to about 20%, preferably about 0.5% to about 15%, and preferably about 1% to about 10%, by weight based on the total weight of the composition.

The pharmaceutical composition according to the present invention may further comprise one or more active pharmaceutical ingredients. The additional active pharmaceutical ingredient may be selected from the group consisting of emtricitabine, elvitegravir, cobicistat, rilpivirine, bictegravir, dolutegravir, darunavir and combinations thereof.

The pharmaceutical composition may comprise a combination of tenofovir alafenamide and emtricitabine. The pharmaceutical composition may comprise a combination of tenofovir alafenamide, emtricitabine and rilpivirine. The pharmaceutical composition may comprise a combination of tenofovir alafenamide, emtricitabine and dolutegravir.

A further embodiment provides a pharmaceutical composition wherein the total quantity of degradation products (e.g. impurities) derived from tenofovir alafenamide is less than about 3%, less than about 2%, less than about 1% after storage for 5 days at 50°C/10% RH in open conditions. Yet a further embodiment provides a pharmaceutical composition wherein the total quantity of degradation products (e.g. impurities) derived from tenofovir alafenamide is less than about 3%, less than about 2%, less than about 1% after storage for 6 months at 25°C/60% RH in open conditions. Yet a further embodiment provides a pharmaceutical composition wherein the total quantity of degradation products (e.g. impurities) derived from tenofovir alafenamide is less than about 3%, less than about 2%, less than about 1% after storage for 6 months at 30°C/65% RH in open conditions. Yet a further embodiment provides a pharmaceutical composition wherein the total quantity of degradation products (e.g. impurities) derived from tenofovir alafenamide is less than about 3%, less than about 2%, less than about 1% after storage for 6 months at 40°C/75% RH in open conditions.

In an embodiment, the pharmaceutical composition according to the present invention may be formulated as a single dosage form.

The pharmaceutical composition may be formulated for oral administration. The composition may be formulated as a tablet such as mono-layered tablets, bilayered tablets, trilayered tablet, multilayer tablet, caplets, minitablets, microtablets, capsules, tablet in tablet, tablets in a capsule, microtablets in a capsule, minitablets in a capsule, granules in a capsule, pellets, pellets in a capsule, powder, granules, extrudes, pellets, beads or spheroids, suspension or any other suitable dosage form meant for oral administration to a patient.

In a preferred embodiment, the pharmaceutical composition according to the present invention may be formulated as a single tablet, and more preferably as a single unit oral tablet. Each tablet may contain polymer-coated tenofovir alafenamide equivalent to 10 mg or 25 mg of tenofovir alafenamide (free base).

The pharmaceutical composition may also be formulated for administration once daily.

The dosage form may comprise: (a) a core comprising a polymer-coated tenofovir alafenamide; and one or more pharmaceutically acceptable excipients, and b) a coating over said core. Any suitable film forming polymer may be used for the coating. Examples of suitable film forming polymers include cellulose acetate, ethyl cellulose, hydroxypropyl methylcellulose, polyacrylic resin and combinations thereof.

The coating may be present in amount of about 1% to about 10%, preferably about 2% to about 8%, and preferably about 2% to about 5%, by weight based on the total weight of the core. In an embodiment, the coating may be present in an amount of about 1%, about 2%, about 3%, about 4%, about 5%, about 6%, about 7%, about 8%, about 9%, or about 10%, by weight based on the total weight of the core. Unless specifically stated otherwise, where the dosage form is coated, it is to be understood that a reference to % weight of the various components of the pharmaceutical composition refers to the amount excluding the coating.

The pharmaceutical composition may be formulated as a granulate composition comprising intragranular components and extragranular components. The granulate composition may include granules comprising polymer-coated tenofovir alafenamide. As used herein, "intragranular components" refers to the ingredients included in the granule. In addition to the granules, the granulate composition may include one or more excipients added to the granules as extragranular components. As used herein, "extragranular components" refers to ingredients added to the as-formed granules.

In an embodiment according to the present invention, the granulate composition may comprise (a) polymer-coated tenofovir alafenamide, (b) a filler, preferably microcrystalline cellulose, (c) a disintegrant, preferably croscarmellose sodium, (d) a lubricant, preferably magnesium stearate and (e) optionally one or more additional active ingredients as the intragranular components. The granulate composition may comprise a lubricant, preferably magnesium stearate as the extragranular component.

In a third aspect, the present invention provides a method of preparing a polymer-coated tenofovir alafenamide comprising the steps of:
a) coating tenofovir alafenamide with a solution comprising a polymer and a solvent; and
b) removing the solvent to obtain the polymer-coated tenofovir alafenamide.

The method of making a polymer-coated tenofovir alafenamide may include mixing the tenofovir alafenamide in a fluid bed granulator together with a solution comprising a polymer and a solvent to form granules comprising the polymer-coated tenofovir alafenamide.

Any suitable solvent may be used in the method. In a preferred embodiment, the solvent may be water, ethanol or a combination thereof, and more preferably purified water or a combination of purified water and ethanol 1:1 (w/w).

The polymer-coated tenofovir alafenamide may be dried by removing the solvent until a target moisture content is reached.

A further embodiment of the present invention may provide a method of preparing a pharmaceutical composition comprising the polymer-coated tenofovir alafenamide made according to the third aspect of the invention.

The method of preparing a pharmaceutical composition may comprise mixing the polymer-coated tenofovir alafenamide made according to the third aspect of the invention with one or more excipients using fluid bed granulation. The one or more excipients may comprise a filler, preferably microcrystalline cellulose, a disintegrant, preferably croscarmellose sodium, a lubricant, preferably magnesium stearate and/or optionally one or more additional active ingredients. In a preferred embodiment, the pharmaceutical composition may be formulated as granules. In such an embodiment, further excipients may be added to the granules as extragranular components.

It has been advantageously found that use of a fluid bed granulation process can not only improve the processability of the composition, but also provide improved tolerance of tenofovir alafenamide to moisture.

The pharmaceutical composition obtained according to the above method may be compressed into a compressed dosage form, e.g. a tablet. For example, the composition may be compressed using a compression pressure of about 50 MPa to about 500 MPa, about 100 MPa to about 400 MPa, about 200 MPa to about 300 MPa, about 100 MPa to about 170 MPa, and about 75 MPa to about 200 MPa. Other suitable compression pressures include about 50, 55, 60, 65, 70 75, 80, 85, 90, 95, 00, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, and 500 MPa.

The compressed dosage form may further be coated with a film coating. For example, tablets may be coated with a film coating. Exemplary coatings include Opadry II Yellow, Opadry II Pink, Opadry II White, Opadry II Grey and Opadry II Purple.

In a fourth aspect, the present invention provides a polymer-coated tenofovir alafenamide produced by a fluid bed granulation process.

The polymer-coated tenofovir alafenamide according to a fourth aspect of the invention may be prepared using the method according to the third aspect of the invention.

In a fifth aspect, the present invention provides a polymer-coated tenofovir alafenamide for use in the prevention and/or treatment of hepatitis B or HIV. The present invention also provides a pharmaceutical composition comprising a polymer-coated tenofovir alafenamide for use in the prevention and/or treatment of hepatitis B or HIV.

In a sixth aspect, the present invention provides a method of preventing and/or treating hepatitis B or HIV, comprising administering an effective amount of a polymer-coated tenofovir alafenamide to a patient in need thereof.

In a seventh aspect, the present invention provides a use of a polymer-coated tenofovir alafenamide in the manufacture of a medicament for the prevention and/or treatment of hepatitis B or HIV.

In accordance with the fifth to seventh aspects of the invention, the polymer-coated tenofovir alafenamide may be used in the prevention and/or treatment of hepatitis B or HIV. The polymer-coated tenofovir alafenamide may be used in the prevention and/or treatment of HIV, preferably HIV-1 or HIV-2.

As used herein, the terms "treating" and "treatment" and like terms refer to the administration of polymer-coated tenofovir alafenamide in an amount effective to prevent, alleviate, or ameliorate one or more symptoms of a disease or condition, i.e., indication, and/or to prolong the survival of the patient being treated.

In some cases, polymer-coated tenofovir alafenamide may be used in combination (for example, in a composition as described herein) with one or more active pharmaceutical ingredients. The additional active pharmaceutical ingredient may be selected from the group consisting of emtricitabine, elvitegravir, cobicistat, rilpivirine, bictegravir, dolutegravir, darunavir and combinations thereof. Specific combinations include:
(a) tenofovir alafenamide and emtricitabine;
(b) tenofovir alafenamide, emtricitabine and rilpivirine; and
(c) tenofovir alafenamide, emtricitabine and dolutegravir.

As used herein, the term "effective amount" indicates that tenofovir alafenamide when administered is sufficient or effective to prevent, alleviate, or ameliorate one or more symptoms of a disease, disorder or medical condition being treated, and/or to prolong the survival of the subject being treated. The therapeutically effective amount will vary depending on the compound, the disease, disorder or condition and its severity and the age, weight, etc., of the patient to be treated.

In an aspect of the present invention, the pharmaceutical composition may be administered as a single dosage form. The composition may also be administered once daily.

The pharmaceutical composition according to the present invention may be administered by oral administration, preferably as a tablet.

The present invention is now described in more detail by, but is not limited to, the following Examples.

### Examples

### Example 1

Exemplary compositions comprising tenofovir alafenamide are set out in Tables 1 and 2 below.

**Table 1: a pharmaceutical composition of tenofovir alafenamide**

| Ingredient | **Amount (mg)** | **Percentage (wt. %)** |
|---|---|---|
| Intragranular ingredients | | |
| Emtricitabine | 200.00 | 52.63 |
| Tenofovir alafenamide free base | 10.00 | 2.63 |
| Methylcellulose (Methocel E5) | 1.76 | 0.46 |
| Microcrystalline cellulose (Avicel PH 200 NF LM) | 134.54 | 35.41 |
| Croscarmellose sodium (Ac-Di-Sol) | 28.00 | 7.37 |
| Magnesium stearate | 1.52 | 0.40 |

| **Extragranular ingredients** | | |
|---|---|---|
| Magnesium stearate | 4.18 | 1.10 |
| Total core: | **380.00** | **100.00** |

| **Coating** | | |
|---|---|---|
| Coating (Opadry II) | 12.00 | - |
| Total: | **392.00** | - |

**Table 2: a pharmaceutical composition of tenofovir alafenamide**

| **Ingredient** | **Amount (mg)** | **Percentage (wt. %)** |
|---|---|---|
| **Intragranular ingredients** | | |
| Emtricitabine | 200.00 | 52.63 |
| Tenofovir alafenamide free base | 25.00 | 6.58 |
| Methylcellulose (Methocel E5) | 4.41 | 1.16 |
| Microcrystalline cellulose (Avicel PH 200 NF LM) | 116.89 | 30.76 |
| Croscarmellose sodium (Ac-Di-Sol) | 28.00 | 7.37 |
| Magnesium stearate | 1.52 | 0.40 |

| **Extragranular ingredients** | | |
|---|---|---|
| Magnesium stearate | 4.18 | 1.10 |
| Total core: | **380.00** | **100.00** |

| **Coating** | | |
|---|---|---|
| Coating (Opadry II) | 12.00 | - |
| Total: | **392.00** | - |

### Example 2

A polymer-coated tenofovir alafenamide was prepared according to the following method.

Tenofovir alafenamide (2.5 kg) was weighed and inserted into a 12 L vessel of a fluid-bed granulator. The coating solution was prepared by dissolving 441 g of Methocel in a mixture of ethanol (4.2 kg) and purified water (4.2 kg). The coating solution was sprayed onto tenofovir alafenamide in a fluid-bed granulator under the following process parameters:
Nozzle: 1 mm
Spraying pressure :2.0 - 2.5 bar
Inlet air temperature: 50 - 60 °C
Product temperature: <35 °C

When spraying was finished, the material was dried (LOD and cooled (<30 °C) in a fluid-bed granulator. The polymer-coated tenofovir alafenamide was then sieved.

### Example 3

Pharmaceutical compositions comprising tenofovir alafenamide with and without a coating were studied by stress tests, and the amount of degradation impurities was measured. Table 3 provides a comparison of the stability test results between tablets comprising non-coated tenofovir alafenamide and tablets comprising coated tenofovir alafenamide. Stability tests of film-coated tablets, packaged in HDPE bottles with desiccant and aluminium sealing, were performed in tempered stability chambers for 6 months.

**Table 3: Results of stability test between tablets comprising coated and non-coated tenofovir alafenamide**

| **Composition** | **Total impurities (%)** | | | |
|---|---|---|---|---|
| | **T0** | **T = 6M (25°C, 60% RH)** | **T = 6M (30°C, 65% RH)** | **T = 6M (40°C, 75% RH)** |
| Emtricitabine/Tenofovir alafenamide 200/10 mg (non-coated tenofovir alafenamide) | 0.25 | 0.89 | 2.58 | 6.89 |
| Emtricitabine/Tenofovir alafenamide 200/10 mg (coated tenofovir alafenamide) | 0.28 | 0.10 | 0.21 | 0.61 |

| | | | | |
|---|---|---|---|---|
| T0: analyzed before stress test at time 0 T = 6M (25°C, 60% RH): stress test 6 months at 25°C and 60% of relative humidity T = 6M (30°C, 65% RH): stress test 6 months at 30°C and 65% of relative humidity T = 6M (40°C, 75% RH): stress test 6 months at 40°C and 75% of relative humidity | | | | |

The results shown in Table 3 demonstrated that the amount of degradation impurities for the non-coated tenofovir alafenamide was significantly higher (0.89 - 6.89 %) compared to the amount of degradation impurities for the coated tenofovir alafenamide (0.10 - 0.61 %) at the same tested conditions.

### Example 4

Example 4 was designed to determine the influence of different weight percentages of the coating on the level of tenofovir degradation. The results are shown in Table 4 below.

**Table 4: Results of stability test of tenofovir alafenamide with different weight percentages of the coating**

| **Composition** | **Total impurities** (%) | | **Increase of impurities (%)** |
|---|---|---|---|
| | **T0** | **T = 5d (50°C, 10% RH)** | |
| Tenofovir alafenamide with 5% coating of Methocel | 0.24 | 0.47 | 0.23 |
| Tenofovir alafenamide with 10% coating of Methocel | 0.27 | 0.38 | 0.11 |
| Tenofovir alafenamide with 15% coating of Methocel | 0.33 | 0.44 | 0.11 |
| Tenofovir alafenamide with 25% coating of Methocel | 0.25 | 0.52 | 0.27 |

| | | | |
|---|---|---|---|
| T0: analyzed before stress test at time 0 T = 5d (50°C, 10% RH): stress test 5 days at 50°C and 20% of relative humidity | | | |

### Example 5

Example 5 was designed to determine the stability of the polymer-coated tenofovir alafenamide by measuring the amount of various impurities.

The stability of the polymer-coated tenofovir alafenamide was tested on two batches sealed with a desiccant in an aluminium foil. The amount of various impurities individually as well as the total impurities content were measured at the beginning of the experiment, and also after three months storage. The results are shown in Table 5 below.

**Table 5: Results of stability test of tenofovir alafenamide showing amounts of impurities**

| **Impurity** | **Limit** | **Initial** | **3 months** |
|---|---|---|---|
| TAF-5 | NMT 0.3% | <0.1% | <0.1% |
| TAF-13 | NMT 0.3% | <0.1% | <0.1% |
| TAF-1 | NMT 0.3% | <0.1% | <0.1% |
| Dimer tenofovir | NMT 0.3% | <0.1% | <0.1% |
| Desphenyl tenofovir alafenamide | NMT 0.3% | <0.1% | <0.1% |
| Unspecified impurities individually | NMT 0.15% | <0.1% | <0.1% |
| **Total Impurities** | NMT 10% | <0.1% | <0.1% |

As shown in Table 5, the amount of all of the impurities individually and also the total impurities content were <0.1%, demonstrating that tenofovir alafenamide coated with a polymer show good stability.

## Claims

1. A polymer-coated tenofovir alafenamide.

2. The polymer-coated tenofovir alafenamide according to claim 1, wherein the tenofovir alafenamide is in a free base form or as a fumarate or hemifumarate salt form, preferably in a free base form.

3. The polymer-coated tenofovir alafenamide according to claim 1 or 2, wherein the polymer is a non-ionizable pharmaceutically acceptable polymer, preferably selected from the group consisting of derivates of cellulose, cellulose ether, polyvinyl alcohol, povidone, shellac, gelatin and combinations thereof.

4. The polymer-coated tenofovir alafenamide according to any one of the preceding claims, wherein the polymer is present in an amount of about 0.5% to about 50%, preferably about 10% to about 20%, by weight based on the total weight of tenofovir alafenamide.

5. The polymer-coated tenofovir alafenamide according to any one of the preceding claims, wherein the weight ratio between the polymer and tenofovir alafenamide is between about 1:200 and about 1:2, preferably between about 1:10 and about 1:5.

6. A pharmaceutical composition comprising the polymer-coated tenofovir alafenamide according to any one of the preceding claims.

7. The pharmaceutical composition according to claim 6, wherein the composition comprises tenofovir alafenamide in an amount of about 1% to about 15% by weight based on the total weight of the composition.

8. The pharmaceutical composition according to claim 6 or 7, wherein the composition comprises about 10 mg to about 30 mg, preferably about 10 mg or 25 mg of tenofovir alafenamide.

9. The pharmaceutical composition according to any one of claims 6 to 8, wherein the composition comprises polymer-coated tenofovir alafenamide in an amount of about 1% to about 60% by weight based on the total weight of the composition.

10. The pharmaceutical composition according to any one of claims 6 to 9, further comprising one or more pharmaceutically acceptable excipients selected from the group consisting of binders, fillers, glidants, lubricants, disintegrants and plasticizers.

11. The pharmaceutical composition according to any one of claims 6 to 10, further comprising one or more active pharmaceutical ingredients selected from the group consisting of emtricitabine, elvitegravir, cobicistat, rilpivirine, bictegravir, dolutegravir, darunavir and combinations thereof.

12. The pharmaceutical composition according to claim 11, wherein the composition comprises a combination of:
(a) tenofovir alafenamide and emtricitabine;
(b) tenofovir alafenamide, emtricitabine and rilpivirine; or
(c) tenofovir alafenamide, emtricitabine and dolutegravir.

13. The polymer-coated tenofovir alafenamide according to any one of claims 1 to 6 or the pharmaceutical composition according to any one of claims 6 to 12, wherein the polymer-coated tenofovir alafenamide is produced by a fluid bed granulation process.

14. The polymer-coated tenofovir alafenamide according to any one of claims 1 to 6 or the pharmaceutical composition according to any one of claims 6 to 12, for use in the prevention and/or treatment of hepatitis B or HIV.

15. A method of preparing a polymer-coated tenofovir alafenamide according to any one of claims 1 to 6, comprising the steps of:
a) coating tenofovir alafenamide with a solution comprising a polymer and a solvent; and
b) removing the solvent to obtain the polymer-coated tenofovir alafenamide.

16. The method according to claim 15, wherein the polymer-coated tenofovir alafenamide is prepared by a fluid bed granulation process.
